# EUROPEAN PATENT APPLICATION

(11) **EP 2 664 325 A1**
(43) Date of publication of application: **20.11.2013**
(21) Application number: 12168319.7
(22) Date of filing: 16.05.2012
(51) Int. Cl.: A61K 31/00, A61K 31/57, A61P 5/24, A61P 15/12, A61P 17/10, A61P 17/14, A61P 17/08, A61P 35/00, A61P 13/08

(54) **Methods for preventing or treating androgen mediated diseases**

(71) Applicant: PregLem S.A., 1228 Plan-les-Ouates Geneva (CH)
(72) Inventor: Pohl, Oliver Martin, 74160 ARCHAMPS (FR); Gotteland, Jean-Pierre, 1205 Geneva (CH)

(57) **Abstract**

The present invention relates generally to a progesterone receptor modulator, or any metabolite thereof for use in the prevention or treatment of androgen mediated diseases characterized in that said progesterone receptor modulator, or any metabolite thereof, is administered to a subject in need thereof.

## Description

### TECHNICAL FIELD

The present invention relates generally to methods for preventing or treating androgen mediated diseases and in particular to a method for the prevention or treatment of Benign prostatic hyperplasia, Premenstrual syndrome, Polycystic ovary syndrome, Prostate cancer, Hirsutism, Acne, Seborrhea , Androgenic alopecia.

### BACKGROUND OF THE INVENTION

Many patients suffering from androgen mediated diseases require intervention in order to improve the diseases' symptoms.

The androgen receptor (AR), a member of the steroid/nuclear receptor superfamily, plays a critical role in normal male development, including the development of the prostate gland.

In addition, AR action plays a fundamental role in the development and progression of conditions such as prostate cancer or benign prostate hyperplasia in men [Hodgson, 2004]. In women, AR receptor related research has revealed that in most cases of Premenstrual Syndrome (PMS) and Premenstrual Dysphoric Disorder (PMDD), these conditions appear to be caused by an inadequate adrenal gland production of progesterone and its metabolite allopregnanolone. [1, 2, 3] Furthermore, excessive androgens, with the low progesterone and allopregnanolone, result in cycle related feelings of irritability, anxiety and agitation seen in PMS/PMDD. [2] Other conditions in women in which the androgen receptor may play a significant role comprise polycysitic ovarian syndrome (PCOS) or hirsutism. Common symptoms of PCOS include menstrual disorders, infertility and high levels of masculinizing hormone;. The most common signs are acne and hirsutism [7]. Approximately three-quarters of patients with PCOS have evidence of hyperandrogenemia [10].

Androgen signaling is mediated through the androgen receptor (AR) and is a nuclear signaling pathway of major importance in mammals. It plays a role in sexual development, maturation and maintenance of sexual function in both males and females. In addition, this hormone signaling pathway affects a large number of non-sexual tissues including, bone, muscle, CNS, liver, etc.

### PMS

Premenstrual syndrome is a common condition affecting a large proportion of women with normal functioning ovaries. Although the cause is still not known, it seems to be related to the fluctuation of oestrogen and progesterone during the ovarian cycles. PMS is also referred to as late luteal phase syndrome (or late luteal phase dysphoric disorder).

The symptoms that occur can be both physical and psychological in nature. Irritability, snappiness and being on a "short fuse", depression and aggression are the most common reported psychological symptoms, but tension and anxiety are also frequent. Other psychological and behavioural symptoms which have been suggested to occur during the premenstrual phase and menstruation include, for example, decreased efficiency, insomnia, confusion, poorer judgment, difficulty in concentrating, crying, loneliness, restlessness, irritability, and mood swings. The effect of these symptoms can be compounded by physical symptoms which vary widely. The most common reported symptoms are tiredness, a feeling of abdominal bloating and breast swelling, and weight gain. Other symptoms which have been suggested to occur include, for example, dizziness, faintness, cold sweats, nausea, vomiting, hot flashes, muscle stiffness, headache, cramps, backache, general aches and pains, and water retention including, for example, weight gain, skin disorders, painful breasts, and swelling.

Altough numerous treatments have been suggested for alleviating or minimizing symptoms of premenstrual syndrome, they don't provide optimal results.

Accordingly, the present invention provides methods for alleviating one or more menstrual symptoms in women associated with premenstrual syndrome, and compositions therefore.

### BPH

Benign prostate hypertrophy is a disease conditioned by age and affects approximately 60% of all men older than 60. It is characterized by an elevated accumulation of dihydrotestosterone in the prostate tissue, said dihydrotestosterone being assumed to cause enlargement of the prostate. The accumulation of dihydrotestosterone is thought to be the result of elevated intracellular bonding based on receptor increase. The increase in receptors is stimulated by the elevation of the estrogen levels relative to androgen levels which decrease with age. The urological symptoms consist in an elevated frequency of miction due to elevated residual urine, which bothers the patients especially during the night hours. This is accompanied by a weak flow of urine, a time-delayed start of miction, and repeated infections of the bladder and kidneys. Surgical elimination of the obstruction due to prostate enlargement is still considered the "gold standard" within the various modalities of treatment. Surgery, however, is not effective for all patients.

Although other alternative treatments have been suggested for alleviating or minimizing symptoms of BPH, they do not provide optimal results. Accordingly, the present invention provides methods for alleviating BPH symptoms.

### PCOS

Polycystic ovary syndrome (PCOS) is one of the most common female endocrine disorders. PCOS produces symptoms in approximately 5% to 10% of women of reproductive age (12-45 years old). It is thought to be one of the leading causes of subfertility and the most frequent endocrine problem in women of reproductive age.

The principal features are anovulation, resulting in irregular menstruation, amenorrhea, ovulation-related infertility, and polycystic ovaries; excessive amounts or effects of androgenic (masculinizing) hormones, resulting in acne and hirsutism; and insulin resistance, often associated with obesity, Type 2 diabetes, and high cholesterol levels.

The symptoms and severity of the syndrome vary greatly among affected women.

Several treatment options are available for the treatment of PCOS among which a medication called metformin to improve the body's sensitivity to insulin. However, although said treatment options exist and allow managing the PCOS symptoms, they are not optimal.

Thus, there remain significant unmet needs for efficient and better long-term therapies for androgen mediated diseases mediated, disease such as BPH, PMS, PCOS, prostate cancer, hirsutism, acne, seborrhea and androgenic alopecia .

### SUMMARY OF THE INVENTION

The present invention relates generally to methods for preventing or treating androgen mediated diseases and in particular to a method for the prevention or treatment of BPH, PMS, PCOS, prostate cancer, hirsutism, acne, seborrhea , androgenic alopecia comprising administering to a subject in need thereof a progesterone receptor modulator, or any metabolite thereof.

In a preferred embodiment, the invention provides methods for preventing or treating androgen mediated diseases and in particular to a method for the prevention or treatment of BPH, PMS, PCOS, prostate cancer, hirsutism, acne, seborrhea , androgenic alopecia comprising administering to a subject in need thereof a selective progesterone receptor modulator CDB-2914 (Ulipristal acetate), or any metabolite thereof.

The present invention also embraces these compounds, pharmaceutically acceptable salts or complexes thereof, pharmaceutical compositions thereof for their use in the preventing or treating androgen mediated diseases such as BPH, PMS, PCOS prostate cancer, hirsutism, acne, seborrhea, androgenic alopecia.

### DETAILED DESCRIPTION OF THE INVENTION

Ulipristal acetate (UPA), a Selective Progesterone Receptor Modulator (SPRM), shows high affinity for the progesterone receptor and a specific in vitro selectivity profile over other nuclear receptor of the same family such as a high affinity for the glucocorticoid receptor, much lower affinity for the androgen receptor and no affinity for the oestrogen receptors.

Surprisingly enough, the Applicants have shown that this specific nuclear receptor selectivity profile confers an unexpected in vivo activity profile with anti-androgenic effects, while no significant anti-glucocorticoid effects .

The Applicants have surpisingly found that seminal vesicles, prostate and epididymidis, in response to ulipristal acetate effect , decrease in weight. This suggests that ulipristal acetetate, because of its specific nuclear receptor profile , is a candidate of choice for the prevention or treatment of androgen mediated diseases and in particular for the prevention or treatment of BPH, PMS, PCOS, prostate cancer, hirsutism, acne, seborrhea , androgenic alopecia.

The present invention (claim1) relates to a progesterone receptor modulator, or any metabolite thereof for use in the prevention or treatment of androgen mediated diseases characterized in that said progesterone receptor modulator, or any metabolite thereof, is administered to a subject in need thereof.

In a further embodiment the present invention relates to the progesterone receptor modulator according to claim 1, wherein the progesterone receptor modulator is a selective progesterone receptor modulator (SPRM), or any metabolite thereof.

In a further embodiment the present invention relates to the progesterone receptor modulator according to any one of the preceding claims, wherein the androgen mediated disease is selected from the list of BPH, PMS, PCOS, prostate cancer, hirsutism, acne, seborrhea and androgenic alopecia.

In a further embodiment the present invention relates to the progesterone receptor modulator according to claim 2 wherein the selective progesterone receptor modulator is CDB-2914 (ulipristal acetate), or any metabolite thereof.

In a further embodiment the present invention relates to the progesterone receptor modulator according to claim 4, wherein CDB-2914 (ulipristal acetate) or any metabolite thereof is administered in a dosage of 1 to 500 mg.

In a further embodiment the present invention relates to the progesterone receptor modulator according to claims anyone of claims 1 to 5, wherein the subject is a mammal, preferably a human being.

In a further embodiment the present invention relates to a kit for preventing or treating androgen mediated diseases selected from the list of BPH, PMS, PCOS, prostate cancer, hirsutism, acne, seborrhea and androgenic alopecia comprising a therapeutically effective amount of a progesterone receptor modulator, a SPRM, or any metabolite thereof optionally with reagents and/or instructions for use.

The present invention also relates to a method for preventing or treating an androgen mediated disease comprising administering a dosage of a progesterone receptor modulator, or any metabolite thereof to a subject in need thereof.

In a further embodiment the present invention relates to a method for preventing or treating an androgen mediated disease comprising administering a dosage of a progesterone receptor modulator, or any metabolite thereof to a subject in need thereof wherein the progesterone receptor modulator is a selective progesterone receptor modulator (SPRM), or any metabolite thereof.

In a further embodiment the present invention relates to a method for preventing or treating an androgen mediated disease, wherein the androgen mediated disease is selected from the list of BPH, PMS, PCOS, prostate cancer, hirsutism, acne, seborrhea and androgenic alopecia.

In a preferred embodiment the selective progesterone receptor modulator to be used according to the method of the invention is CDB-2914 (ulipristal acetate), or any metabolite thereof.

In another embodiment, CDB-2914 (ulipristal acetate) or any metabolite thereof is administered in a dosage of 1 to 500 mg.

"Administering", as it applies in the present invention, refers to contact of a therapeutically effective amount of a progesterone receptor modulator, a SPRM, or an active metabolite thereof, to the subj ect.

Usually, the "subject" is well-recognized in the art, and, is used herein to refer to a mammal and, more preferably, a human being.

The term "comprise" or "comprising" is generally used in the sense of include/including, that is to say permitting the presence of one or more features or components. Additionally, the term "comprising" also encompasses the term "consisting".

As used in the specification and claims, the singular form "a", "an" and "the" include plural references unless the context clearly dictates otherwise.

As used herein, "at least one" means "one or more."

Throughout the specification (description and claims) and for the ease of reading, the terms "progesterone receptor modulator", "selective progesterone receptor modulator (SPRM)", and "metabolite thereof", refer also to the salts of said respective progesterone receptor modulator, selective progesterone receptor modulator or metabolite thereof.

This invention also envisages the use of an PRM, SPRM e.g. ulipristal acetate, or a metabolite thereof, in a pharmaceutically acceptable salt form. Examples of such salts may include sodium, potassium, calcium, aluminum, gold and silver salts. Also contemplated are salts formed with pharmaceutically acceptable amines such as ammonia, alkyl amines, hydroxyalkylamines, N-methylglucamine and the like. Certain basic compounds also form pharmaceutically acceptable salts, e.g., acid addition salts. For example, pyrido-nitrogen atoms may form salts with strong acid, while compounds having basic substituents such as amino groups also form salts with weaker acids. Examples of suitable acids for salt formation are hydrochloric, sulfuric, phosphoric, acetic, citric, oxalic, malonic, salicylic, malic, fumaric, succinic, ascorbic, maleic, pamoic, methanesulfonic and other mineral and carboxylic acids well known to those skilled in the art. The salts are prepared by contacting the free base form with a sufficient amount of the desired acid to produce a salt in the conventional manner. The free base forms may be regenerated by treating the salt with a suitable dilute aqueous base solution such as dilute aqueous NaOH, potassium carbonate, ammonia and sodium bicarbonate. The free base forms differ from their respective salt forms somewhat in certain physical properties, such as solubility in polar solvents, but the acid and base salts are otherwise equivalent to their respective free base forms for purposes of the invention.

All such acid and base salts are intended to be pharmaceutically acceptable salts within the scope of the invention and all acid and base salts are considered equivalent to the free forms of the corresponding compounds for purposes of the invention.

Active metabolites of ulipristal acetate, or of a salt thereof, may be identified using routine techniques known in the art and their activities determined using tests such as those described herein. Such metabolites may result for example from the oxidation, reduction, hydrolysis, amidation, deamidation, esterification, deesterification, enzymatic cleavage, and the like, of the administered ulipristal acetate or of a salt thereof. Accordingly, the invention includes active metabolites of ulipristal acetate or of a salt thereof, including compounds produced by a process comprising contacting a compound of this invention with a mammal for a period of time sufficient to yield a metabolic product thereof. Such metabolite may also be produced in vitro by oxidation, reduction, hydrolysis, amidation, deamidation, esterification, deesterification, or enzymatic cleavage of the corresponding ulipristal or salt thereof. Examples of metabolites of ulipristal acetate (CDB-2914), include those described in Attardi et al, 2004, e.g. monodemethylated CDB-2914 (CDB-3877); didemethylated CDB-2914 (CDB-3963) ; 17alpha-hydroxy CDB-2914 (CDB-3236); aromatic A-ring derivative of CDB-2914 (CDB-4183). Preferably the amount of progesterone receptor modulator, SPRM, or a metabolite thereof is effective to for preventing or treating androgen mediated diseases in a mammal, without clinically significant antiglucocorticoid activity.

The progesterone receptor modulator, the SPRM, or a metabolite thereof, may be administered by any convenient route, including oral, buccal, sublingual, parenteral, transdermal, vaginal, rectal, etc. For a brief review of present methods for drug delivery, see, Langer, Science 249:1527- 1533 (1990), which is incorporated herein by reference. Methods for preparing administrable compounds are known or are apparent to those skilled in the art and are described in more detail in, for example, Remington's Pharmaceutical Science, 17th Ed., Mack Publishing Company, Easton, Pa. (1985), which is incorporated herein by reference, and which is hereinafter referred to as "Remington."

For solid compositions, conventional nontoxic solid carriers may be used which include, for example, pharmaceutical grades of mannitol, lactose, starch, magnesium stearate, sodium saccharine, talcum, cellulose, glucose, sucrose. For oral administration, a pharmaceutically acceptable nontoxic composition is formed by incorporating any of the normally employed excipients, such as those carriers previously listed.

The mode of administration possibilities include tablets, capsules, lozenges, pills, transdermal patches, dental pastes, suppositories, inhalants, solutions, ointments , parenteral depots, vaginal rings, vaginal gels and intra-uterine delivery systems.

The oral and vaginal routes are preferred.

Oral solid dosage forms are preferentially compressed tablets or capsules. Compressed tablets may contain diluents to increase the bulk of the progesterone receptor modulator, the SPRM, or a metabolite thereof, so that production of a compressed tablet of practical size is possible. Binders, which are agents which impart cohesive qualities to powdered materials may be also necessary. Povidone, starch, gelatin, sugars such as lactose or dextrose, and natural and synthetic gums may be used. Disintegrants are generally necessary in the tablets to facilitate break-up of the tablet. Disintegrants include starches, clays, celluloses, algins, gums and cross-linked polymers. Lastly small amounts of materials known as lubricants and glidants are included in the tablets to prevent adhesion of the tablet material to surfaces in the manufacturing process and to improve the flow characteristics of the powder material during manufacture. Colloidal silicon dioxide is most commonly used as a glidant and compounds such as talc, magnesium stearate or stearic acids are most commonly used as lubricants. Procedures for the production and manufacture of compressed tablets are well known by those skilled in the art (See Remington). Capsules are solid dosage forms using preferentially either a hard or soft gelatin shell as a container for the mixture of the progestogen agent or progesterone receptor modulator and inert ingredients. Procedures for production and manufacture of hard gelatin and soft elastic capsules are well known in the art (See Remington).

In cases where the progesterone receptor modulator, a SPRM, or a metabolite thereof, is included in a solution, the formulation may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, among others.

Useful intranasal formulations of a progesterone receptor modulator, a SPRM, or a metabolite thereof may contain at least one stabilizer and/or one surfactant. Among the pharmaceutically acceptable surfactants are polyoxyethylene castor oil derivatives, such as polyoxyethylene-glycerol- triricinoleate, also known as polyoxyl 35 caster oil (CREMOPHOR EL), or poloxyl 40 hydrogenated castor oil (CREMOPHOR RH40) both available from BASF Corp.; mono-fatty acid esters of polyoxyethylene (20) sorbitan, such as polyoxyethylene (20) sorbitan monolaurate (TWEEN 80), polyoxyethylene monostearate (TWEEN 60), polyoxyethylene (20) sorbitan monopalmitate (TWEEN 40), or polyoxyethylene 20 sorbitan monolaurate (TWEEN 20) (all available from ICI Surfactants of Wilmington, Del.); polyglyceryl esters, such as polyglyceryl oleate; and polyoxyethylated kernel oil (LABRAFIL, available from Gattefosse Corp.). Preferably, the surfactant will be between about 0.01 % and 10% by weight of the pharmaceutical composition. Among the pharmaceutically useful stabilizers are antioxidants such as sodium sulfite, sodium metabisulfite, sodium thiosulfate, sodium formaldehyde sulfoxylate, sulfur dioxide, ascorbic acid, isoascorbic acid, thioglycerol, thioglycolic acid, cysteine hydrochloride, acetyl cysteine, ascorbyl palmitate, hydroquinone, propyl gallate, nordihydroguaiaretic acid, butylated hydroxytoluene, butylated hydroxyanisole, alpha-tocopherol and lecithin. Preferably, the stabilizer will be between about 0.01% and 5% by weight of the pharmaceutical composition. Suspensions may also include chelating agents such as ethylene diamine tetraacetic acid, its derivatives and salts thereof, dihydroxyethyl glycine, citric acid and tartaric acid among others. Additionally, proper fluidity of a suspension can be maintained, for example, by the use of coating materials such as lecithin, by the maintenance of the required particle size in the case of dispersions and by the use of surfactants, such as those previously mentioned. Solid dosage forms for oral administration include capsules, tablets, pills, powders and granules. In such solid dosage forms, the active compound may be mixed with at least one inert, pharmaceutically acceptable excipient or carrier, such as sodium citrate or dicalcium phosphate and/or (a) fillers or extenders such as starches, lactose, sucrose, glucose, mannitol and silicic acid; (b) binders such as carboxymethylcellulose, alginates, gelatin, polyvinylpyrrolidone, sucrose and acacia; (c) humectants such as glycerol; (d) disintegrating agents such as agar-agar, calcium carbonate, potato or tapioca starch, alginic acid, certain silicates and sodium carbonate; (e) solution retarding agents such as paraffin; (f) absorption accelerators such as quaternary ammonium compounds; (g) wetting agents such as cetyl alcohol and glycerol monostearate;(h) absorbents such as kaolin and bentonite clay; and (i) lubricants such as talc, calcium stearate, magnesium stearate, solid polyethylene glycols, sodium lauryl sulfate and mixtures thereof. In the case of capsules, tablets and pills, the dosage form may also comprise buffering agents.

Alternatively, or additionally, it will become apparent that ulipristal acetate, or a metabolite thereof, may be administered alone or in combination with other treatments, therapeutics or agents, either simultaneously or sequentially dependent upon the condition to be treated.

The present invention also contemplates a kit for preventing or treating an androgen mediated disease comprising a progesterone receptor modulator, or any metabolite thereof, optionally with reagents and/or instructions for use.

Generally, the Kit comprises a container and a label or package insert on or associated with the container. Suitable containers include, for example, bottles, vials, syringes, etc. The containers may be formed from a variety of materials such as glass or plastic. The container holds the progesterone receptor modulator, or any metabolite thereof of the invention which is effective for preventing or treating androgen mediated diseases. The label or package insert indicates that the composition is used for treating the condition of the invention.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein.

Various references are cited throughout this specification, each of which is incorporated herein by reference in its entirety.

The foregoing description will be more fully understood with reference to the following Examples. Such Examples, are, however, exemplary of methods of practicing the present invention and are not intended to limit the scope of the invention.

### EXAMPLES

### Example 1: Ulipristal acetate anti-androgenic effect in a 4 week oral toxicity study in mice.

### Material and Methods

During a 104 week rat carcinogenicity assay, we observed seminal vesicles and prostate contraction at dose-levels of 1 (seminal vesicles, only), 3 and 10mg/kg/day (PGL09-005). Similarly, during 4 and 26 week assays in the mouse, decreases in epididymidis weights were recorded at dose-levels ranging between 15 and 300mg/kg/day (tables 1 and 2).

**Table 1: 4 week oral toxicity study with UPA in mice - organ weights**

| Test Article-related Organ Weight Changes - Terminal Male and Female (Percent change relative to control) | | | | | | |
|---|---|---|---|---|---|---|
| Dose level: mg/kg/day | 20 | | 80 | | 300 | |
| Sex | M | F | M | F | M | F |
| Number Examined | 10 | 10 | 10 | 10 | 10 | 10 |
| Epididymides (g) | □4.8 | NA | □9.6 | NA | □26.5^{b} | NA |
| Epididymides/BWt | □4.6 | NA | □12.4 | NA | □28.1^{b} | NA |
| Epididymides/BrWt | □4.2 | NA | □8.7 | NA | □23.9^{b} | NA |
| ^{a} Significantly different from control;(p<0.05) | | ↑ - | Increased | | NC - No Change | |
| | | ↓ - | Decreased | | NA - Not Applicable | |
| ^{b} Significantly different from control; (p<0.01) | | M - | Male | | | |
| | | F - | Female | | | |
| BWt - Body Weight | | | | | | |
| BrWt - Brain Weight | | | | | | |

### Results:

These toxicity studies show that seminal vesicles, prostate and epididymidis are androgen responsive organs. The decrease in organ weight under the UPA effect is suggestive of anti-androgenic like effects.

### Example 2: Ulipristal acetate anti-androgenic effect in a 26 week oral carcinogenicity study in mice.

### Material and Methods

**Table 2: 26 week oral carcinogenicity study with UPA in mice - organ weights**

| Test Article-related Organ Weight Changes - Terminal Male and Female (Percent change relative to vehicle control) | | | | | | |
|---|---|---|---|---|---|---|
| Dose level: mg/kg/day | 15 | | 45 | | 130 | |
| Sex | M | F | M | F | M | F |
| Number Examined | 23 | 22 | 24 | 25 | 25 | 22 |
| Epididymides (g) | □3.00 | NA | □7.00 | NA | □10.00^{a} | NA |
| Epididymides/BWt% | □4.15 | NA | □6.48 | NA | □8.18 | NA |
| Epididymides/BrWt ratio | □2.72 | NA | □7.34 | NA | □8.79 | NA |
| ^{a} Significantly different from control;(p<0.05) | | ↑ - | Increased | | NC - No Change | |
| | | ↓ - | Decreased | | NA - Not | |
| ^{b} Significantly different from control; (p<0.01) | | Applicable | | | | |
| | | M - | Male | | | |
| BWt - Body Weight | | F - | Female | | | |
| BrWt - Brain Weight | | | | | | |

### Results:

These toxicity studies show that seminal vesicles, prostate and epididymidis are androgen responsive organs. The decrease in organ weight under the UPA effect is suggestive of anti-androgenic like effects.

### References:

[1] Lombardi I, Luisi S, Quirici B, Monteleone P, Bernardi F, Liut M, Casarosa E, Palumbo M, Petraglia F, Genazzani AR. Adrenal response to adrenocorticotropic hormone stimulation in patients with premenstrual syndrome. Gynecol Endocrinol. 2004 Feb; 18(2):79-87. PMID: 15195499
[2] Eriksson E, Sundblad C, Lisjo P, Modigh K, Andersch B. Serum levels of androgens are higher in women with premenstrual irritability and dysphoria than in controls. Psychoneuroendocrinology. 1992 May-Jul;17(2-3):195-204. PMID: 1438645
[3] Monteleone P, Luisi S, Tonetti A, Bernardi F, Genazzani AD, Luisi M, Petraglia F, Genazzani AR. Allopregnanolone concentrations and premenstrual syndrome. Eur J Endocrinol. 2000 Mar; 142(3):269-73. PMID: 10700721
7 H Teede; A Deeks; L Moran (30 June 2010). "Polycystic ovary syndrome: a complex condition with psychological, reproductive and metabolic manifestations that impacts on health across the lifespan". BMC Medicine (BioMedCentral) 8: 41. doi:10.1186/1741-7015-8-41. Retrieved 14 November 2011.
10 Huang A, Brennan K, Azziz R (April 2010). "Prevalence of hyperandrogenemia in the polycystic ovary syndrome diagnosed by the National Institutes of Health 1990 criteria". Fertil. Steril. 93 (6): 1938-41. doi:10.1016/j.fertnstert.2008.12.138. PMC 2859983. PMID 19249030.

## Claims

1. A progesterone receptor modulator, or any metabolite thereof for use in the prevention or treatment of androgen mediated diseases **characterized in that** said progesterone receptor modulator, or any metabolite thereof, is administered to a subject in need thereof.

2. The progesterone receptor modulator according to claim 1, wherein the progesterone receptor modulator is a selective progesterone receptor modulator (SPRM), or any metabolite thereof.

3. The progesterone receptor modulator according to any one of the preceding claims, wherein the androgen mediated disease is selected from the list of BPH, PMS, PCOS, prostate cancer, hirsutism, acne, seborrhea and androgenic alopecia.

4. The progesterone receptor modulator according to claim 2 wherein the selective progesterone receptor modulator is CDB-2914 (ulipristal acetate), or any metabolite thereof.

5. The progesterone receptor modulator according to claim 4, wherein CDB-2914 (ulipristal acetate) or any metabolite thereof is administered in a dosage of 1 to 500 mg.

6. The progesterone receptor modulator according to claims anyone of claims 1 to 5, wherein the subject is a mammal, preferably a human being.

7. A kit for preventing or treating androgen mediated diseases selected from the list of BPH, PMS, PCOS, prostate cancer, hirsutism, acne, seborrhea and androgenic alopecia comprising a therapeutically effective amount of a progesterone receptor modulator, a SPRM, or any metabolite thereof optionally with reagents and/or instructions for use.
